(19) Europäisches Patentamt
European Patent Office
Office européen des brevets

(11) EP 2 560 984 B1

(12) # EUROPEAN PATENT SPECIFICATION

(45) Date of publication and mention of the grant of the patent:
**01.04.2015 Bulletin 2015/14**

(21) Application number: **11724024.2**

(22) Date of filing: **13.04.2011**

(51) Int Cl.:
***C07J 41/00*** *(2006.01)*

(86) International application number:
**PCT/IB2011/000825**

(87) International publication number:
**WO 2011/132045 (27.10.2011 Gazette 2011/43)**

(54) **PROCESS FOR THE PREPARATION OF DIENOGEST SUBSTANTIALLY FREE OF IMPURITIES**
VERFAHREN ZUR HERSTELLUNG VON WEITGEHEND KONTAMINATIONSFREIEM DIENOGEST
PROCÉDÉ DE PRÉPARATION DE DIENOGEST SENSIBLEMENT DÉPOURVU D'IMPURETÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.04.2010 IN KA04382010**

(43) Date of publication of application:
**27.02.2013 Bulletin 2013/09**

(73) Proprietor: **Lupin Limited**
**State of Maharashtra, Mumbai 400 098 (IN)**

(72) Inventors:
- **TAMBE, Suhas, Ganpat**
  **411 042 Pune (IN)**
- **KIRANGE, Bhushan, Bhanudas**
  **411 042 Pune (IN)**
- **SINGH, Gurvinder, Pal**
  **411 042 Pune (IN)**
- **RAY, Purna, Chandra**
  **411 042 Pune (IN)**

(74) Representative: **Srinivasan, Ravi Chandran**
**J A Kemp**
**14 South Square**
**Gray's Inn**
**London WC1R 5JJ (GB)**

(56) References cited:
**EP-A2- 1 935 898 DD-A5- 296 495**

- **CHENG QILU ET AL: "SYNTHESIS OF 17.ALPHA.-CYANOMETHYL-17.BETA.-HYDROXY-4,9 (10)-ESTRADIE N-3-ONE AND ITS 4,9(10),11-TRIENE DERIVATIVE", YIYAO GONGYE - PHARMACEUTICAL INDUSTRY, SHANGHAI, CN, vol. 16, no. 9, 1 January 1985 (1985-01-01), pages 399-402, XP008077887, ISSN: 0255-7223 cited in the application**
- **MENZENBACH B ET AL: "SYNTHESE POTENTIELLER METABOLITEN DER STS 557 (DIENOGEST). TEIL3: 17ALPHA-HYDROXYMETYL-17BETA-HYDROXYESTRA-4 , 9-DIEN-3-ON", DIE PHARMAZIE, GOVI VERLAG PHARMAZEUTISCHER VERLAG GMBH, ESCHBORN, DE, vol. 39, no. 7, 1 July 1984 (1984-07-01), page 496/497, XP002009736, ISSN: 0031-7144**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 560 984 B1

## Description

### FIELD OF THE INVENTION

The present invention is related to dienogest that is substantially free of impurities and process for its preparation.

### BACKGROUND OF THE INVENTION

Dienogest is an orally active synthetic progesterone (or progestin), used as an oral contraceptive in combination of ethinylestradiol. It has antiandrogenic activity that can improve androgenic symptoms. It is a non-ethinylated progestin which is structurally related to testosterone.

Dienogest is chemically known as, (17α)-17-Hydroxy-3-oxo-19-norpregna-4,9-diene-21-nitrile or 17α-Cyanomethyl-17β-hydroxy-4,9-estradien-3-one and it is represented by following structure:

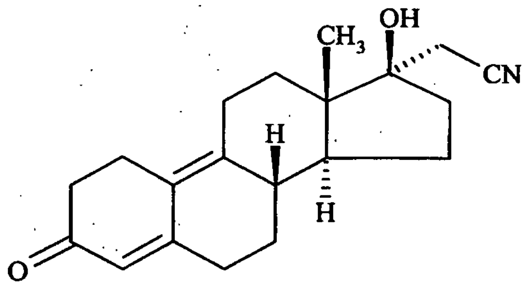

I

Dienogest

The patent US 4248790, describe process for preparation of dienogest as in Scheme-I.

**Scheme-I**

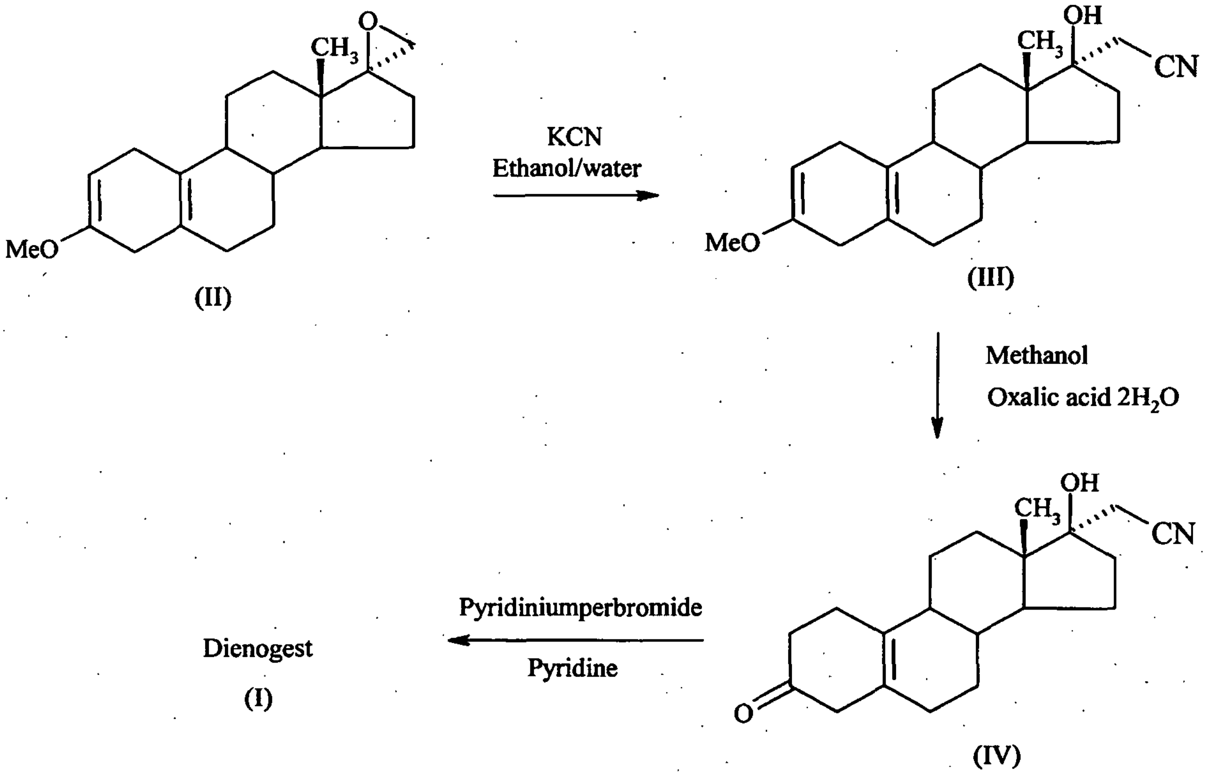


(II) (III) (IV)

Dienogest (I)

The publication Zhang Xiuping et al.; Pharmaceutical Industry (1985), 16(9), 399-401, describe the conversion of ketal (3,3-dimethoxy-estra-5(10), 9(11)-diene-17-one) (V) to dienogest (I) in three steps as shown in Scheme-II, wherein ethylene oxide intermediate (VI) is reacted with alcohol solution of potassium cyanide to produce (VII) followed by acid hydrolysis.

**Scheme - II**

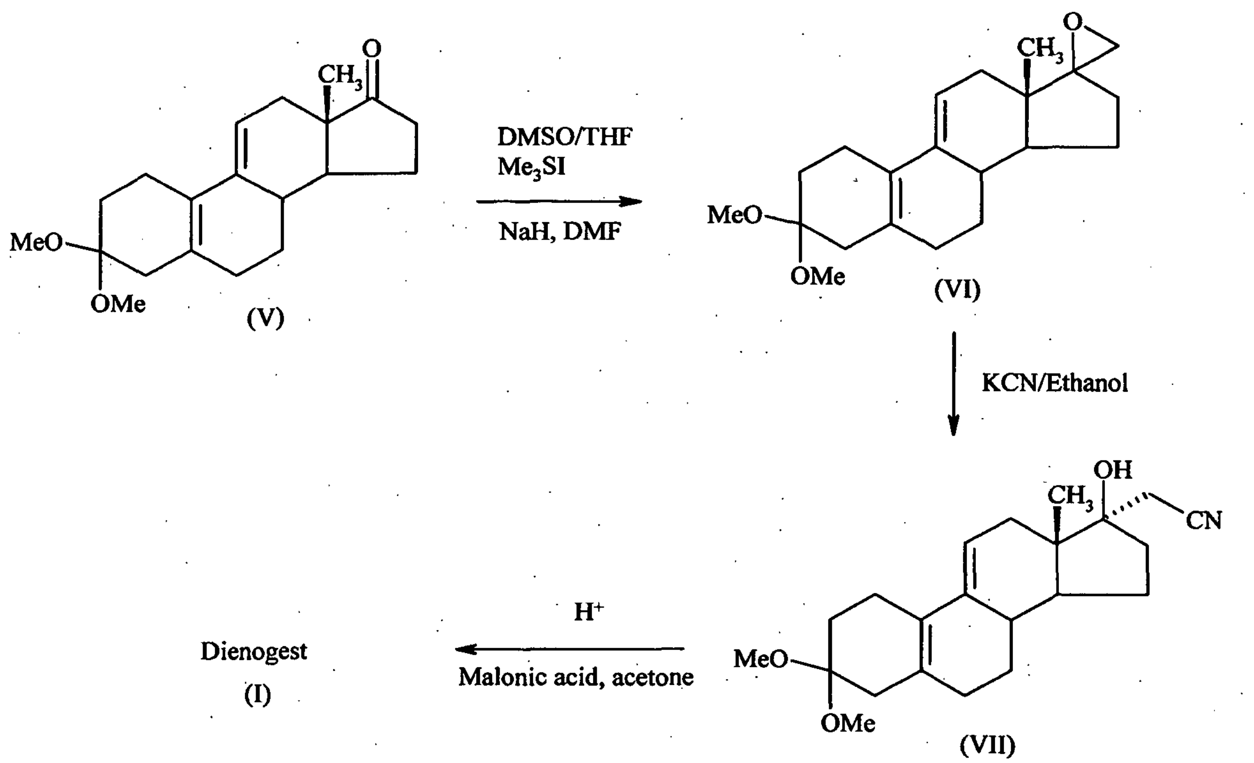


The patent EP 1935898 disclose preparation of dienogest (I) in which the ketal (V) is reacted with $CNCH_2CeCl_2$, followed by treatment with acetic acid and hydrochloric acid.

The German patent application DD 296,495 describes a one-pot synthesis of dienogest from 3,3-dimethoxy-estra-5(10), 9(11)-diene-17-one (V), on treatment with dilute sulfuric acid.

Prior art processes described above use toxic reagents like trimethyl sulfonium iodide or alkali cyanides. Removal of residual cyanide demands additional operations of destruction of cyanide which makes it a costly method.

Several methods are known in the literature for purification of dienogest by crystallization. These are discussed below.

The patent US 4248790, discloses crystallization of dienogest from ethyl acetate and 80% acetonitrile. The publication Zhang Xiuping et al., Pharmaceutical Industry (1985), 16(9), 399-401, and patent DD 205170 disclose crystallization of dienogest from methanol. EP 1935898 and US 5955622 disclose crystallization of dienogest from acetone.

The patent US 20080287404 A1 covers recrystallisation of dienogest from ethyl acetate, acetone, tert-butyl methyl ether, diisopropyl ether, acetonitrile, methanol, ethanol or aqueous mixtures of different ratio of these solvents.

The patent EP 1963354 B1 discloses purification of crude dienogest by preparative HPLC to obtain pure dienogest with total amount of impurities up to maximum 0.1 % and individual impurities up to maximum 0.02 % (by HPLC). This method has the disadvantage that it is difficult on production levels.

The patents US 5438134 and EP 231671 discloses compound 17α-cyanomethyl-17β-hydroxy-13β-methyl-gona-5(10),9(11)-diene-3-one, which is referred herein as diene impurity.

The diene impurity is also mentioned as 19-Norpregna-5(10),9(11)-diene-21-nitrile, 17-hydroxy-3-oxo-, (17α).

It is always desirable to prepare pharmaceutical products of high purity having a minimum amount of impurities, in order to reduce adverse side effects and to improve the shelf life of active ingredient, as well as its formulation. In some cases, it has been found that high purity also facilitates in formulation process.

The present invention is directed to provide an improved process for the preparation of dienogest having minimum amount of impurities.

## OBJECTIVE OF THE INVENTION

The objective of present invention is to provide dienogest that is substantially free of impurities and a novel process for its preparation.

Another objective of the present invention is to provide novel method of purification of dienogest by crystallization from mixture of dimethylformamide and water.

## SUMMARY OF THE INVENTION

The present invention provides dienogest containing diene impurity, (17α-cyanomethyl-17β-hydroxy-5,9-estradien-3-one) less than 0.05 % and other total impurities less than about 0.1% (area percentage by HPLC analysis).

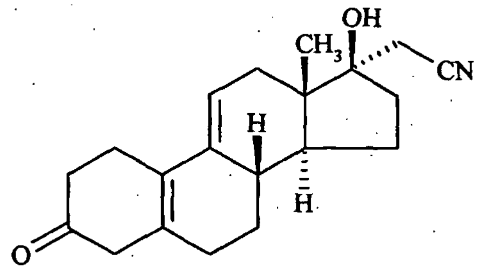

**Diene Impurity**

The present invention also relates to a novel process for preparing dienogest with a minimum amount of impurities.

The present invention further provides novel process of preparing dienogest which involves deprotection of ketal 3,3-dimethoxy-17α-cyanomethyl-17β-hydroxy-estra-5(10),9(11)-diene (VII) with perchloric acid.

A purification method by crystallization of dienogest from dimethylformamide - water mixture is also described as a preferred embodiment.

## DETAILED DESCRIPTION

The present invention provides dienogest substantially free of impurities. In particular, the present invention provides dienogest having diene impurity less than 0.05% and other total impurities less than 0.1 %, (measured as area percentage by HPLC).

The present invention further relates to a novel process for preparing dienogest which is substantially free of its impurities.

The conversion of ketal, 3,3-dimethoxy-17α-cyanomethyl-17β-hydroxy-estra-5(10),9(11)-diene (VII) by treatment with hydrochloric acid, sulfuric acid, acetic acid or oxalic acid to obtain dienogest is already known in the literature. However when these acids were used, it was observed that, the shift of the double bonds into conjugation with 3-keto group was incomplete. This resulted in the formation of dienogest (I) along with diene impurity in various amounts depending on the acid employed, which was difficult to remove by purification.

In order to control formation of diene impurity, a detailed investigation of reaction of ketal (VII) with various strong and weak acids was carried out. The observations of this study are provided in Table-1.

When strong acids such as hydrobromic acid, triflic acid, p-toluene sulphonic acid and trifluoroacetic acid were used, same difficulty was encountered; the shifting of the double bonds into conjugation with 3-keto group did not go to completion, resulting in the formation of diene impurity.

The use of weaker acids like oxalic acid led to very less shifting of double bonds and eventually gave diene impurity, as major product.

**Table-1:** Study of deprotection of ketal (VII) by using different acids at 20 - 30°C

| S. No. | Acid | Solvent | Dienogest (I) (%)# | Diene impurity (%)# |
|---|---|---|---|---|
| 1 | Conc. HCl | Acetonitrile | 91.13 | 8.1 |
| 2 | Hydrobromic acid | Acetonitrile | 78.05 | 19.43 |
| 3 | 30 % sulfuric acid | Acetonitrile | 74.19 | 25.55 |

(continued)

| S. No. | Acid | Solvent | Dienogest (I) (%)# | Diene impurity (%)# |
|---|---|---|---|---|
| 4 | p-Toluene sulfonic acid. $H_2O$ | Acetonitrile | 91.97 | 6.43 |
| 5 | Trifluoroacetic acid | Acetonitrile | * | * |
| 6 | Triflic acid | Acetonitrile | 89.32 | 5.4 |
| 7 | Oxalic acid.$2H_2O$ | Methanol | 0.07 | 98.01 |
| 8 | Perchloric acid (70 %) | Acetic acid | 93.75 | 2.84 |
| 9 | Perchloric acid (70 %) | Ethyl acetate | 97.46 | 1.5 |
| 10 | Perchloric acid (70 %) | Acetonitrile | 99.49 | 0.15 |
| 11 | Perchloric acid (70 %) | Acetonitrile | 99.29 | 0.12 |
| # area percentage by HPLC<br>* degradation observed in TLC, HPLC data was not generated | | | | |

Dienogest (I) >99% + Diene Impurity ≤ 0.15% ← Perchloric acid / acetonitrile — (VII) — HCl or sulfuric acid / acetonitrile → Dienogest (I) 74-91% + Diene Impurity 8-25.5%

Conversion of ketal (VII) to dienogest, with perchloric acid was studied in different solvents such as acetic acid, ethyl acetate and acetonitrile. It was surprisingly found that, with perchloric acid, deprotection followed by shift of double bonds into conjugation with 3-keto group was almost complete to yield dienogest with minimum impurities (see entry 8 - 11 in Table-1). In acetic acid and ethyl acetate the diene impurity was slightly higher than in acetonitrile as solvent. Thus, the inventors found that perchloric acid in acetonitrile as solvent was the most suitable for conversion of ketal (VII) to dienogest (I) which provided dienogest of purity up to 99.49 % and diene impurity less than or equal to 0.15 %.

The conversion of ketal (VII) to dienogest with perchloric acid can be carried out at temperature ranging from 10 to 50°C, preferably 20 - 30°C.

Other solvents like propyl acetate, butyl acetate, ethyl propionate, propionic acid, propiononitrile, butyronitrile, dimethyl acetamide, dimethyl formamide, tetrahydrofuran, dioxane, acetone, methyl isobutyl ketone, methylethyl ketone; and mixtures thereof are also useful for conversion of ketal (VII) to dienogest, with perchloric acid.

In another aspect, the present invention provides as a preferred embodiment a method for purification of dienogest by crystallization from dimethylformamide (DMF) - water mixture. The purification afforded dienogest wherein the level of diene impurity was up to 0.02 % and other total impurities less than 0.1 %.

The advantage of the current invention is that the process is environment friendly as it does not use toxic reagents like trimethyl sulfonium iodide or alkali cyanides.

The dienogest prepared by the process of present invention is preferred for pharmaceutical formulation, since it is substantially free of impurities.

## SPECIFIC EXAMPLES

The present invention can be illustrated in one of its embodiments by the following nonlimiting examples.

The purity of dienogest was measured as area percentage by HPLC with Zorbax Eclipse XDB, C-8 (4.6 x 150 mm), 5 μm as column, Water:Acetonitrile (90:10) and (10:90) as mobile phases at flow rate 1.0 mL/min, and UV detection at 240 nm.

The compound 3,3-dimethoxy-estra-5(10), 9(11)-diene-17-one (V) was prepared from estra-4,6-diene-3,17-dione as described in the publication "Menzenbach, B et al., Pharmazie (1984), 39(7), 496-7".

**Example 1:** 3,3-dimethoxy-17α-cyanomethyl-17β-hydroxy-estra-5(10),9(11)-diene

A mixture of n-hexyl lithium (33% solution in hexane), (180 g) and tetrahydrofuran (300 mL) was cooled to -50 to -40°C. A mixture of acetonitrile (33 mL) and tetrahydrofuran (200 mL) was added to it. A solution of 3,3-dimethoxy-estra-5(10), 9(11)-diene-17-one (V) (100 g, 0.32 mole) in tetrahydrofuran (600 mL) was added to the mixture at -50 to -40°C and stirred for 90 minutes. After completion of reaction, temperature raised to -10 to 0°C and water was added.

Organic layer was separated, washed with brine and concentrated under vacuum. Residue was taken in acetonitrile, cooled to 0-5°C, stirred for one hour, filtered and dried under reduced pressure at 35-40°C to give 100 g of title compound.

**Example 2:** Preparation of crude Dienogest

The compound (100 g) of example-1 in acetonitrile (600 mL) was cooled to 0-5°C and 70% perchloric acid (150 mL) was added and stirred for one hour at 20-30°C. After completion of reaction, water (1500 mL) was added and solid was filtered to obtain crude product. (HPLC data: Dienogest - 99.55 %, diene impurity- 0.125 %)

**Example 3:** Purification of crude Dienogest

a) Wet solid from example-2 was charcoalised in dimethylformamide (400 mL) at 45-50°C for one hour, filtered, washed with dimethylformamide (100ml) and to the combined filtrate water (200 ml) was added. Reaction mass stirred for 2 hours at 0-5°C. Solid was filtered and dried under reduced pressure at 40-45°C to give 66 g crude dienogest. (HPLC data: Dienogest - 99.79 %, Diene impurity - 0.02 %).

b) Dienogest (25 g) from example 3(a) was taken im dimethylformamide (125 mL) and heated to 40-50°C, filtered, washed with dimethylformamide (25 ml) and to the combined filtrate water (37.5 ml) was added to precipitate the product, cooled to 0 to 5°C and stirred for 2 hours. Solid obtained was filtered, washed with water and dried under vacuum at 40-45°C to give 23 g of Dienogest.(HPLC data: Dienogest - 99.9 %, Diene impurity - 0.02 %).

## Claims

**1.** A process for preparation of dienogest of formula (I)

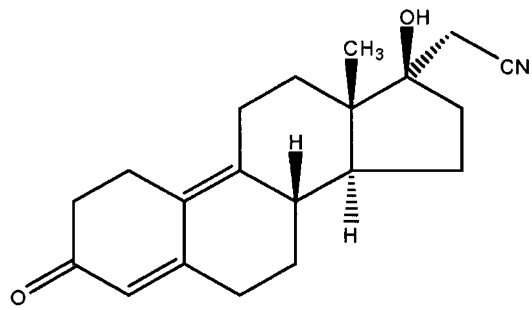

(I)

comprising reaction of 3,3-dimethoxy-17α-cyanomethyl-17β-hydroxy-estra-5(10),9(11)-diene (VII)

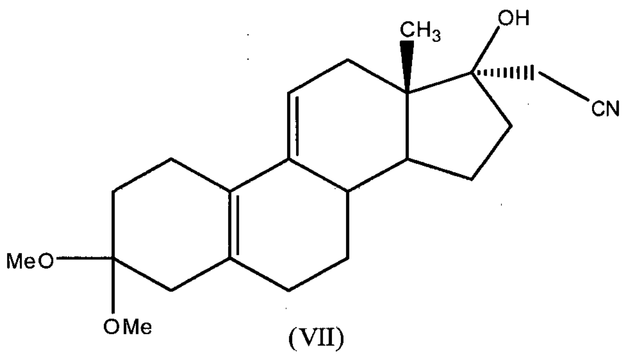

(VII)

with perchloric acid.

2. The process according to claim 1, wherein the reaction is carried out in the presence of solvent selected from acetonitrile, acetic acid, ethyl acetate, propyl acetate, butyl acetate, ethyl propionate, propionic acid, propiononitrile, butyronitrile, dimethyl acetamide, dimethyl formamide, tetrahydrofuran, dioxane, acetone, methyl isobutyl ketone, methylethyl ketone and mixtures thereof.

3. The process according to claim 2, wherein the solvent is acetonitrile.

4. The process according to claim 1, wherein dienogest has the diene impurity

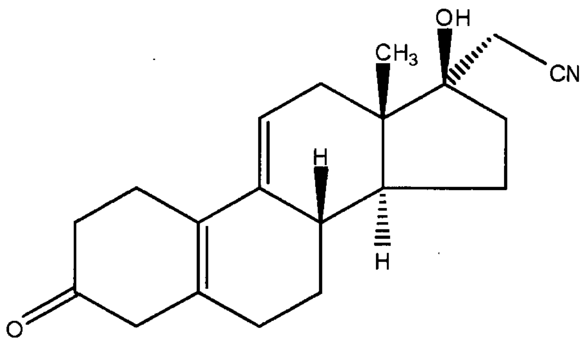

Diene Impurity

less than or equal to 0.15% and total impurities less than or equal to 0.5% measured as area percentage by HPLC.

5. The process according to claim 1, further comprising purification of dienogest (I) by crystallisation from mixture of dimethylformamide and water.

6. The process according to claim 5, wherein dienogest has total impurities less than or equal to 0.25% measured as area percentage by HPLC.

7. The process according to claim 5, wherein dienogest has total impurities less than or equal to 0.1% measured as area percentage by HPLC.

8. The process according to claim 5, wherein dienogest has diene impurity less than or equal to 0.05% measured as area percentage by HPLC.

9. The process according to claim 5, wherein dienogest has diene impurity les than or equal to 0.02% measured as area percentage by HPLC.

**Patentansprüche**

1. Verfahren zur Herstellung von Dienogest der Formel (I)

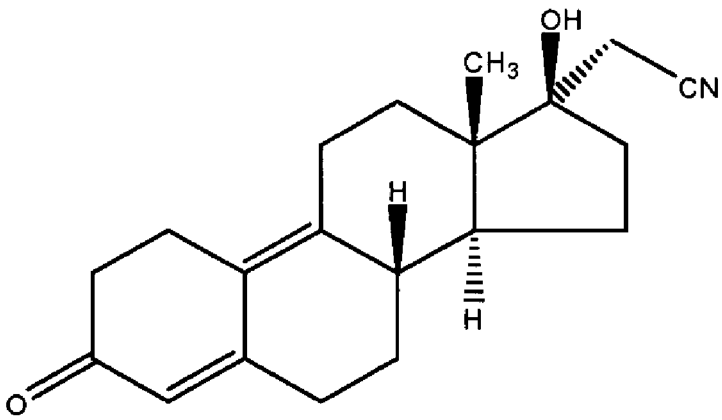

(I)

umfassend die Umsetzung von 3,3-Dimethoxy-17α-cyanomethyl-17β-hydroxy-estra-5(10),9(11)-dien (VII)

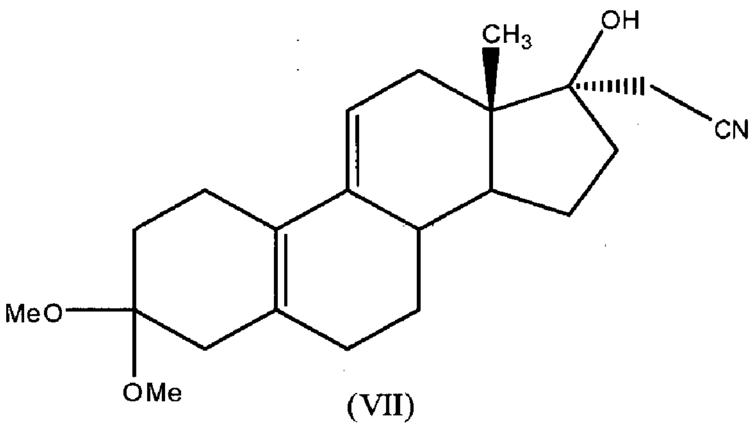

(VII)

mit Perchlorsäure.

2. Verfahren nach Anspruch 1, wobei die Umsetzung ausgeführt wird in Gegenwart eines Lösungsmittels, ausgewählt aus Acetonitril, Essigsäure, Ethylacetat, Propylacetat, Butylacetat, Ethylpropionat, Propionsäure, Propionnitril, Butyronitril, Dimethylacetamid, Dimethylformamid, Tetrahydrofuran, Dioxan, Aceton, Methylisobutylketon, Methylethylketon und Gemischen davon.

3. Verfahren nach Anspruch 2, wobei das Lösungsmittel Acetonitril ist.

4. Verfahren nach Anspruch 1, wobei Dienogest die Dien-Verunreinigung

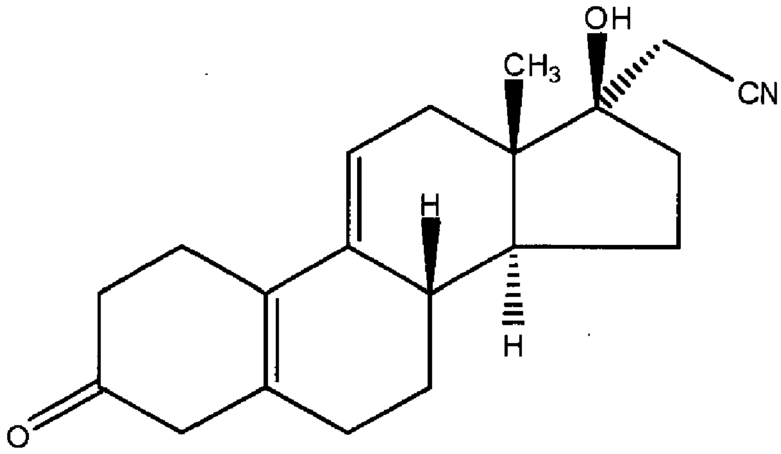

Dien-Verunreinigung

von weniger als oder gleich 0,15% aufweist und Gesamtverunreinigungen von weniger als oder gleich 0,5% aufweist, gemessen als Flächenprozente mittels HPLC.

5. Verfahren nach Anspruch 1, des weiteren umfassend die Reinigung von Dienogest (I) durch Umkristallisation aus einem Gemisch von Dimethylformamid und Wasser.

6. Verfahren nach Anspruch 5, wobei Dienogest Gesamtverunreinigungen von weniger als oder gleich 0,25% aufweist, gemessen als Flächenprozente mittels HPLC.

7. Verfahren nach Anspruch 5, wobei Dienogest Gesamtverunreinigungen von weniger als oder gleich 0,1% aufweist, gemessen als Flächenprozente mittels HPLC.

8. Verfahren nach Anspruch 5, wobei Dienogest Dien-Verunreinigung von weniger als oder gleich 0,05% aufweist, gemessen als Flächenprozente mittels HPLC.

9. Verfahren nach Anspruch 5, wobei Dienogest Dien-Verunreinigung von weniger als oder gleich 0,02% aufweist, gemessen als Flächenprozente mittels HPLC.

## Revendications

1. Procédé de préparation du diénogest, de formule (I) :

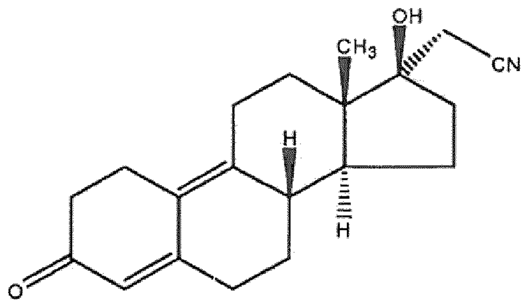

(I)

qui comporte le fait de faire réagir du 3,3-diméthoxy-17α-(cyano-méthyl)-17β-hydroxy-oestra-5(10),9(11)-diène, de formule (VII)

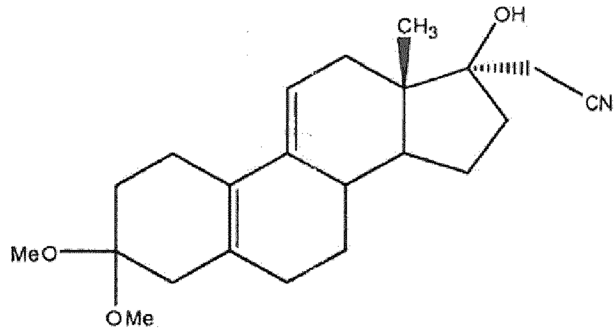

(VII)

avec de l'acide perchlorique.

2. Procédé conforme à la revendication 1, dans lequel la réaction est réalisée en présence d'un solvant choisi parmi les acétonitrile, acide acétique, acétate d'éthyle, acétate de propyle, acétate de butyle, propionate d'éthyle, acide propionique, propiononitrile, butyronitrile, diméthyl-acétamide, diméthyl-formamide, tétrahydrofurane, dioxane, acétone, méthyl-isobutyl-cétone, méthyl-éthyl-cétone, et les mélanges de ces solvants.

3. Procédé conforme à la revendication 2, dans lequel le solvant est de l'acétonitrile.

4. Procédé conforme à la revendication 1, dans lequel le diénogest contient de l'impureté diénique de formule :

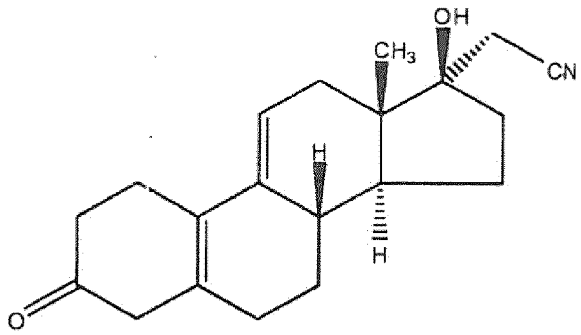

(impureté diénique)

en une teneur inférieure ou égale à 0,15 %, et des impuretés en une teneur totale inférieure ou égale à 0,5 %, teneurs mesurées en pourcentage d'aire par CLHP.

5. Procédé conforme à la revendication 1, qui comporte en outre le fait de purifier le diénogest de formule (I) par cristallisation, dans un mélange de diméthyl-formamide et d'eau.

6. Procédé conforme à la revendication 5, par lequel le diénogest présente une teneur totale en impuretés inférieure ou égale à 0,25 %, teneur mesurée en pourcentage d'aire par CLHP.

7. Procédé conforme à la revendication 5, par lequel le diénogest présente une teneur totale en impuretés inférieure ou égale à 0,1 %, teneur mesurée en pourcentage d'aire par CLHP.

8. Procédé conforme à la revendication 5, par lequel le diénogest présente une teneur en impureté diénique inférieure ou égale à 0,05 %, teneur mesurée en pourcentage d'aire par CLHP.

9. Procédé conforme à la revendication 5, par lequel le diénogest présente une teneur en impureté diénique inférieure ou égale à 0,02 %, teneur mesurée en pourcentage d'aire par CLHP.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4248790 A
- EP 1935898 A
- DD 296495
- DD 205170
- US 5955622 A
- US 20080287404 A1
- EP 1963354 B1
- US 5438134 A
- EP 231671 A


### Non-patent literature cited in the description


- **ZHANG XIUPING et al.** *Pharmaceutical Industry,* 1985, vol. 16 (9), 399-401
- **MENZENBACH, B et al.** *Pharmazie,* 1984, vol. 39 (7), 496-7